# EUROPEAN PATENT APPLICATION

(11) **EP 4 821 445 A1**
(43) Date of publication of application: **14.10.2026**
(21) Application number: 23960875.5
(22) Date of filing: 22.12.2023
(51) Int. Cl.: A61B 8/13

(54) **METHOD FOR IMAGING BODY TISSUE RAPIDLY AND ROBUSTLY USING WAVEFORM INVERSION FROM ULTRASOUND EXAMINATION DATA**

(30) Priority: 05.12.2023 JP 2023205720
(71) Applicant: JGI, Inc., Tokyo 112-0012 (JP); Université Grenoble Alpes, 38400 Saint Martin d'Hères (FR)
(72) Inventor: KATOU Masafumi, Tokyo 112-0012 (JP); TERANISHI Keisuke, Tokyo 112-0012 (JP); HIKIDA Akira, Tokyo 112-0012 (JP); ASAKAWA Eiichi, Tokyo 112-0012 (JP); Romain Brossier, 38400 Saint Martin dHeres (FR); Ludovic Metivier, 38400 Saint Martin dHeres (FR); Jean Virieux, 38400 Saint Martin dHeres (FR); Pengliang Yang, 38400 Saint Martin dHeres (FR)
(74) Representative: Weinstein Services & Conseils
(86) International application number: PCT/JP2023/046287
(87) International publication number: WO 2025/120868

(57) **Abstract**

A method is provided for fast and robust imaging of body tissue from ultrasound examination data using full-waveform inversion. Ultrasound data are acquired, and a single wavefield is generated using multiple computing units operating in parallel. Squared errors between observed and simulated waveforms are integrated across all units to compute a cost function. The update gradient and pseudo-Hessian matrix are repartitioned in the x- and y-directions to match CPU allocation, enabling efficient smoothing and model updating. A quasi-Newton method is applied to determine the step length for updating acoustic-wave-velocity and viscous-attenuation (Q-value) parameters. By repeating these operations, high-resolution and robust imaging of body tissue is achieved with computation times comparable to those of existing diagnostic systems such as X-ray CT or MRI.

## Description

### Technical Field

The present invention relates to full-waveform inversion that images internal body tissue based on acoustic wave propagation velocity and attenuation Q-value, rather than producing conventional echo images of acoustic reflectors and scatterers from data acquired by an ultrasound medical device. The present invention provides a method for enabling high-speed numerical computation that is essential for the practical implementation of full-waveform inversion on ultrasound examination data.

### Background Art

Full-waveform inversion is a subsurface visualization technique based on wave theory developed in the field of seismology (see Non-Patent Documents 1 and 2 below). In principle, full-waveform inversion can be applied to data acquired by ultrasound diagnostic equipment, and several patents have disclosed such applications in combination with ultrasound devices (see Patent Documents 1-6 below).

Ultrasound imaging is preferable to X-ray imaging, CT scans, and mammography examinations, all of which use X-rays, because ultrasound involves no risk of radiation exposure. A typical example of ultrasound diagnostic equipment is an ultrasound imaging device (ultrasound scanner), a system capable of providing real-time images. However, ultrasound examinations require skilled physicians or technicians to repeatedly perform scanning in order to identify problematic areas.

Mammography, a typical breast cancer screening method, has difficulty distinguishing between dense fibroglandular tissue and cancerous tissue in women with dense breasts. Since full-waveform inversion enables imaging of body tissue based on acoustic wave propagation velocity and attenuation Q-value, it becomes possible to numerically distinguish between dense fibroglandular tissue and cancerous tissue, even in women with dense breasts.

Although MRI has slightly lower resolution than X-ray CT, it involves no radiation exposure, and the computational cost is low enough for the imaging results to be obtained on the same day.

As described in Patent Document 5 listed below, full-waveform inversion must provide real-time imaging in order to achieve performance comparable to MRI. To enable such real-time imaging, the turnaround time must be reduced by at least an order of magnitude.

In addition, when using data in a limited frequency band-particularly in highfrequency bands that require propagation over several wavelengths relative to the size of the imaging target-full-waveform inversion exhibits strong dependence on the initial model, making it difficult to achieve robustness.

Although time-domain full-waveform inversion significantly improves robustness compared to frequency-domain methods, it has not been sufficiently explored due to its extremely high computational cost.

For the reasons described above, full-waveform inversion has not yet been put into practical use as an ultrasound-based tissue imaging technique.

### Prior-art Document

### Patent Documents

Patent Document 1: Japanese Unexamined Patent Application Publication No. 2020-18789
Patent Document 2: Japanese Unexamined Patent Application Publication No. 2021-19839
Patent Document 3: Japanese Unexamined Patent Application Publication No. 2018-528829
Patent Document 4: Japanese Unexamined Patent Application Publication No. 2020-501648
Patent Document 5: Japanese Unexamined Patent Application Publication No. 2020-501735
Patent Document 6: International Publication No. WO 2013/116854

### Non-Patent Documents

Non-Patent Document 1: Lailly, P., "The seismic inverse problem as a sequence of before-stack migrations," Conference on Inverse Scattering: Theory and Applications, Society for Industrial and Applied Mathematics (SIAM), 1983, pp. 206-220.
Non-Patent Document 2: Tarantola, A., "Inversion of seismic reflection data in the acoustic approximation," Geophysics, 49(8), 1984, pp. 1259-1266.
Non-Patent Document 3: Kato, M., "A study on numerical simulation based on viscoelastic wave theory of train-induced ground vibrations," Ph.D. Dissertation, Graduate School of Engineering, Kyoto University, 2008.
Non-Patent Document 4: Virieux, J., "P-SV wave propagation in heterogeneous media: Velocity-stress finite-difference method," Geophysics, 51, 1986, pp. 889-901.
Non-Patent Document 5: Yang, P., et al., "Wavefield reconstruction in attenuating media: A checkpointing-assisted reverse-forward simulation method," Geophysics, 81(6), 2016, pp. R349-R362.
Non-Patent Document 6: Yang, P., et al., "A review on the systematic formulation of 3-D multiparameter full waveform inversion in viscoelastic medium," Geophysical Journal International, 207, 2016, pp. 129-149.
Non-Patent Document 7: Shin, C., et al., "Improved amplitude preservation for prestack depth migration by inverse scattering theory," Geophysical Prospecting, 49(5), 2001, pp. 592-606.

### Summary of the Invention

### Technical Problem

The object of the present invention is to provide a method that enables fast and robust imaging of body tissue from ultrasound examination data using full-waveform inversion, addressing the issues inherent in the conventional techniques described above.

### Solution to Problem

First, to improve the robustness of full-waveform inversion, a time-domain computational approach is adopted. Full-waveform inversion in the time domain requires an image pixel size of at least 30 pixels per wavelength. The adoption of the time-domain computational approach increases the computational time but provides higher imaging resolution.

Instead of the acoustic wave equation cited in previous patents, a viscous acoustic wave equation is employed. This enables the imaging results to include viscous attenuation Q-values in addition to acoustic wave propagation velocity.

Next, to accelerate the computation, parallel processing is performed on a large number of computing units. Because each computing unit can be composed of general-purpose CPUs, the costs can be kept low even when many computing units are used simultaneously. However, because full-waveform inversion requires a very large memory space, the memory space must be partitioned. In other words, accelerating full-waveform inversion is achieved by incorporating a memory-space partitioning method into the inversion process, as described below.
(1) When solving the wave equation (for both forward and backward propagation), the memory space is partitioned evenly in the x- and y-directions. In the partitioned memory space, the wave equation for the initial model is solved using the finite-difference method. At each time step of the finite-difference method, data are exchanged at the boundaries of the partitioned domains. At the same time, the partial cost functions, obtained from the residuals between the synthetic data and the observed data in each domain, are gathered from the partitioned memory space and summed into a single cost function.
(2) The update gradient and the pseudo-Hessian matrix are calculated while keeping the memory space partitioned as in step (1).
(3) Gaussian smoothing is applied in the y-direction to the update gradient and the pseudo-Hessian matrix. In this case, the memory space is partitioned evenly only in the x-direction.
(4) After step (3), Gaussian smoothing is applied in the x-direction to the update gradient and the pseudo-Hessian matrix. In this case, the memory space is partitioned evenly only in the y-direction.
(5) The update gradient is preconditioned by the pseudo-Hessian matrix while keeping the memory space partitioned evenly in the y-direction as in step (4).
(6) The norm of the update gradient is calculated while keeping the memory space partitioned evenly in the y-direction as in step (4), and the domain-wise partial norms are gathered from the partitioned memory space and summed into a single norm.
(7) The update direction vector is calculated from the norm obtained in step (6), and the update gradient is scaled by this direction vector to obtain the update to the initial model. The obtained update amount is applied to the initial model to obtain the updated model.
(8) The partitioned memory space is restored to the configuration of step (1), and steps (1) through (7) are repeated.
(9) When the cost function converges to a value below a predetermined threshold, or when the number of iterations reaches a preset upper limit, the updating process is terminated and the final imaging result is obtained.

That is, the method for imaging body tissue from ultrasound examination data using full-waveform inversion includes the following steps:
(a) acquiring ultrasound examination data;
(b) generating a single wavefield using a plurality of computing units (CPUs);
(c) integrating, across all of the CPUs, squared residuals between the waveforms recorded at receiving points from the wavefield and the observed waveforms acquired in step (a), the squared residuals corresponding to fc in Equation (5) below;
(d) repartitioning an update gradient and a pseudo-Hessian matrix in the x-direction into domains evenly assigned to the CPUs;
(e) repartitioning the domains that were evenly partitioned in the x-direction into domains evenly assigned to the CPUs in the y-direction;
(f) calculating a step length necessary for a model update in full-waveform inversion by a quasi-Newton method, while keeping the domains partitioned in the x-direction; and
(g) performing imaging of a viscous attenuation Q-value in addition to an acoustic wave propagation velocity by repeating steps (b) through (f).
Equation (5) ${f}_{c} = 1 / \left(2Δt\right) {\sum }_{s = 1}^{Ns} {\sum }_{r = 1}^{Nr} {\sum }_{t = 1}^{Nt} {\left[d_obs\left(s, r, t\right)-d_syn\left(s, r, t\right)\right]}^{2}$ (5) where *fc*, a scalar quantity, is the cost function defined as the sum of the squared residuals between the observed data (*d_obs*) and the synthetic data (*d_syn*) calculated from the initial model, taken over all receiving points and all time samples, and where *Ns*, *Nr*, and *Nt* denote, respectively, the number of source (transmitting) points, the number of receiving points, and the number of time samples.

### Effects of the Invention

The present invention provides a method for fast and robust imaging of body tissue from ultrasound examination data by full-waveform inversion.

### Brief Description of the Drawings

FIG. 1 is a diagram showing the arrangement of transmitting/receiving (T/R) points.
FIG. 2A is a diagram illustrating the numerical model used in the present invention. FIG. 2B is a diagram illustrating experimentally derived physical properties of body tissue, showing the relationship between viscous attenuation Q-value and acoustic wave propagation velocity V.
FIG. 3A is a diagram illustrating a wavelet waveform. FIG. 3B is a diagram illustrating the frequency spectrum corresponding to the wavelet waveform of FIG. 3A.
FIG. 4 is a diagram illustrating the arrangement of a staggered grid.
FIG. 5 is a snapshot of the wavefield taken 64 µs after the transmission time.
FIGS. 6A to 6E illustrate examples of domain partitioning (i.e., partitioning of the computational memory space). FIG. 6A shows no partitioning; FIG. 6B shows partitioning into two subdomains; FIG. 6C into four subdomains; FIG. 6D into eight subdomains; and FIG. 6E into sixteen subdomains.
FIG. 7 is a diagram illustrating data exchange between CPUs that are adjacent to each other in the x direction.
FIG. 8 is a diagram illustrating data exchange between CPUs that are adjacent to each other in the y direction.
FIG. 9 is a diagram illustrating the observed data.
FIG. 10A illustrates the acoustic wave propagation velocity V of the initial model used for full-waveform inversion. FIG. 10B illustrates the viscous attenuation Q-value of the initial model. FIG. 10C illustrates the medium density ρ of the initial model.
FIG. 11 is a diagram showing synthetic data computed using the initial model.
FIG. 12 is a diagram showing the residuals between the observed data and the synthetic data.
FIG. 13 is a snapshot of the wavefield obtained by back-propagating the residuals, taken 64 µs after the transmission time.
FIG. 14A is a diagram illustrating the update gradient for ρV². FIG. 14B is a diagram illustrating the update gradient for Q⁻¹.
FIGS. 15A to 15C are diagrams illustrating methods for partitioning the computational domain. FIG. 15A illustrates a four-way division of the transmitting points 1 to N. FIG. 15B illustrates partitioning of the computational domain in the x-direction. FIG. 15C illustrates partitioning of the computational domain in the y-direction.
FIG. 16A is an image showing the acoustic wave propagation velocity V. FIG. 16B is an image showing the viscous attenuation Q-value.
FIG. 17 is a graph of the cost function as a function of iteration number *k*.
FIG. 18 is a set of diagrams illustrating the model updating process.
FIG. 19 is a flowchart illustrating the full-waveform inversion model-updating process with parallel computation.
FIG. 20 is a benchmark graph illustrating computation time (solid line) and memory usage (dashed line) as functions of the number of domain divisions.
FIGS. 21A and 21B are snapshot diagrams of frequency-domain wavefields, where FIG. 21A shows forward propagation and FIG. 21B shows back propagation.
FIGS. 22A and 22B are diagrams illustrating the update gradients in the frequency domain, where FIG. 22A shows the gradient for the acoustic wave propagation velocity V, and FIG. 22B shows the gradient for the viscous attenuation Q-value.
FIG. 23 is a flowchart illustrating the imaging method according to the present invention, in which the imaging result (acoustic wave propagation velocity and viscous attenuation Q-value) is obtained by performing full waveform inversion in the time domain using parallel computation.

### Description of the Embodiments

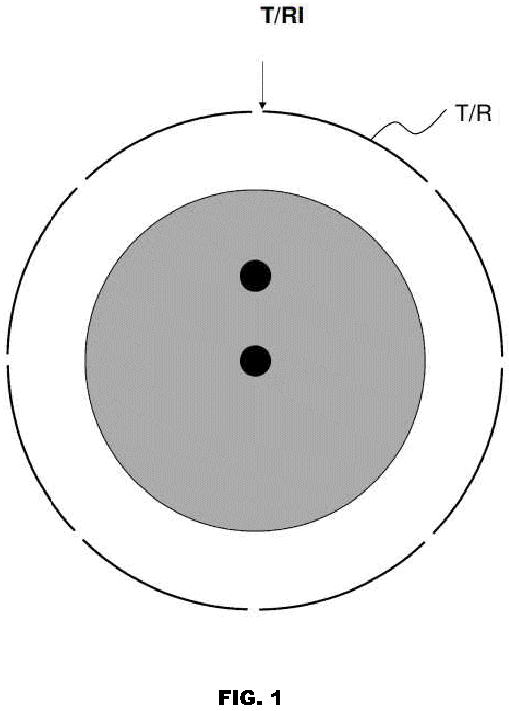

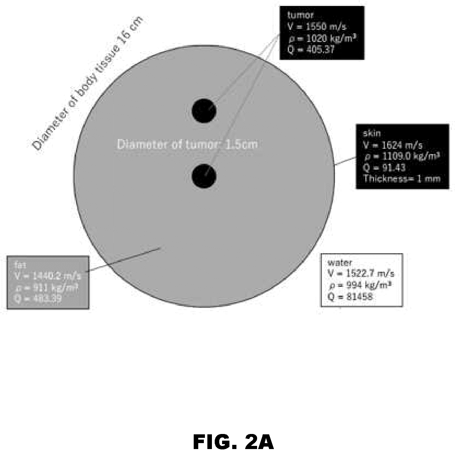

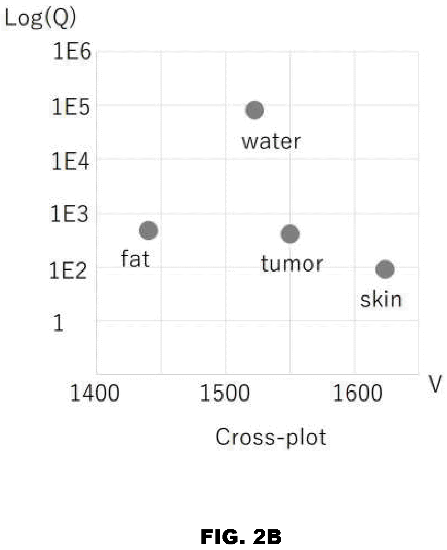

To illustrate the embodiment, numerical simulations are performed using the numerical model and the arrangement of piezoelectric elements shown in FIG. 1. FIGS. 2A and 2B illustrate the physical parameters of body tissue derived from experiments. The acoustic wave propagation velocities of water and tumor tissue are similar, whereas their viscous attenuation Q-values differ significantly. This suggests that when tumors are located in body tissue with high water content, such as blood vessels or mammary glands, imaging based solely on acoustic wave propagation velocity makes tumor detection difficult. In such cases, imaging of the viscous attenuation Q-value plays a significant role in detecting tumors.

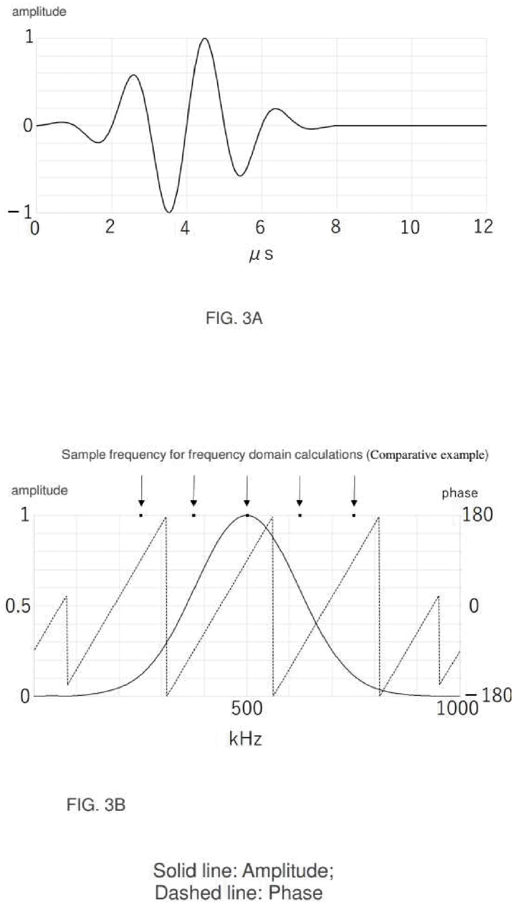

FIG. 3A illustrates the waveform of the ultrasonic wavelet, and FIG. 3B illustrates the corresponding frequency spectrum. The ultrasonic signal shown in FIGS. 3A and 3B is a narrow-band signal of approximately two octaves or less, which can be generated by a general-purpose piezoelectric element. In the example of FIG. 2, the usable frequency range is roughly 200 kHz to 800 kHz, although the bandwidth may be selected as desired depending on the application.

In FIG. 1, the geometry is configured such that the body tissue is surrounded by eight segment groups, each including 256 piezoelectric elements, resulting in a total of 2,048 piezoelectric elements arranged in a clockwise manner. From a practical standpoint, a certain gap is present between adjacent segments, and no data can be acquired within these gap intervals. The radius is 11.7 cm, the receiver spacing is 0.336 mm, and the gaps between segments range from 5.71 mm to 6.38 mm.

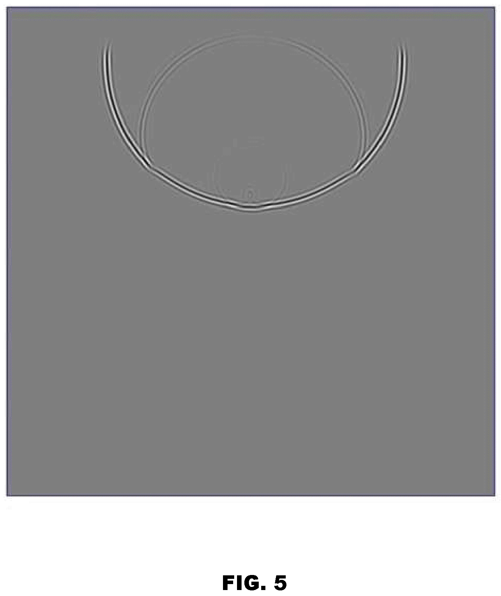

In order to describe the full-waveform inversion procedure, a case is assumed where an ultrasonic signal with the waveform shown in FIG. 5 is transmitted from the piezoelectric element indicated by the arrow (T/R1) in FIG. 1.

The pressure wave emitted from the piezoelectric element is computed as a total wavefield consisting of sound pressure (*σ*) and displacement velocities (*vₓ, v_{y}*). This is achieved by solving, using the finite-difference method, the acoustic wave equation with viscosity, which is expressed as a set of four coupled differential equations: Equation (1), which represents Hooke's law, Equation (2), which defines the memory variable (y), and Equations (3) and (4), which are the equations of motion.
Equation (1) $\dot{σ} = {ρV}^{2} \left(\frac{\partial {v}_{x}}{\partial x}+\frac{\partial {v}_{y}}{\partial y}\right) + {\sum }_{l = 1}^{L} {γ}_{l}$ (1) Equation (2) ${\dot{γ}}_{l} = \frac{ρ {V}^{2}}{{ω}_{l} \left(1+{\sum }_{i = 1}^{L} {τ}_{i}\right)} \left(\frac{\partial {v}_{x}}{\partial x}+\frac{\partial {v}_{y}}{\partial y}\right) + {γ}_{l} , \left(l=1,2,…,L\right)$ (2) Equation (3) ${\dot{v}}_{x} = \frac{1}{ρ} \frac{\partial σ}{\partial x}$ (3) Equation (4) ${\dot{v}}_{y} = \frac{1}{ρ} \frac{\partial σ}{\partial y}$ (4)

The superscript "·" denotes differentiation with respect to time. The parameters p and V represent the density of the medium and the acoustic wave propagation velocity, respectively. The variables *σ*, *vₓ,* and *v_{y}* denote the sound pressure, the displacement velocity in the x-direction, and the displacement velocity in the y-direction, respectively. L is the number of relaxation mechanisms, which is assumed to be three. The exact derivation and the method for converting the L frequencies (ω₁), which define the viscous Q value, into the corresponding relaxation times (τ₁) are described in Non-Patent Document 3.

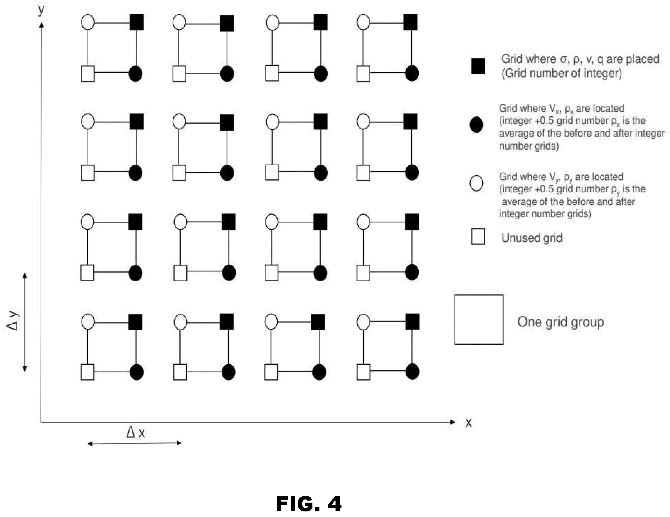

The finite-difference method is a technique for solving the above differential equations (1)-(4) by directly discretizing them, i.e., replacing the spatial derivative ∂x with Δx and the time derivative ∂t with Δt. The staggered-grid arrangement shown in FIG. 4 is employed to improve the numerical accuracy of the finite-difference method. The staggered-grid arrangement was originally proposed by J. Virieux, as described in Non-Patent Document 4. The discretization accuracy is set to fourth order in space and second order in time.

FIG. 5 is a snapshot of the sound-pressure field taken 64 µs after the transmission time.

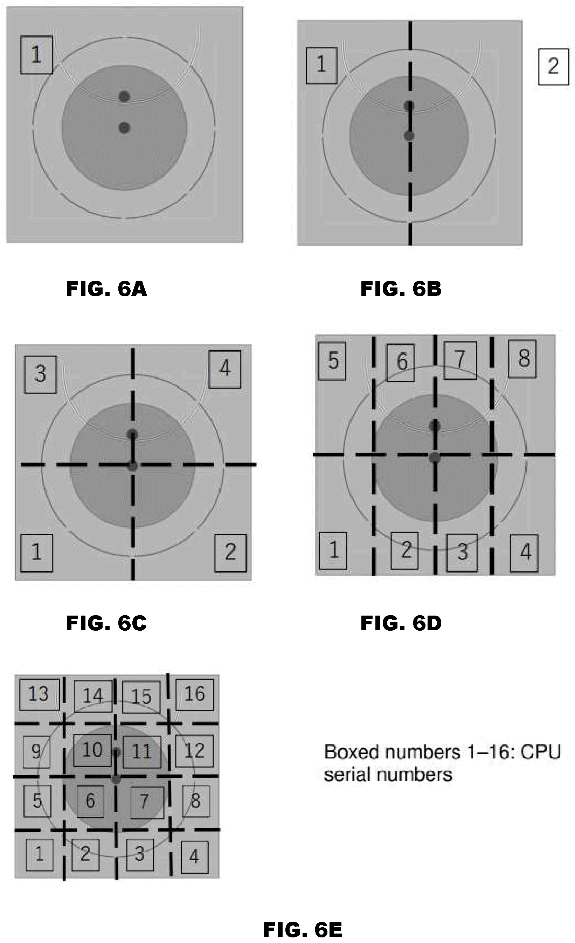

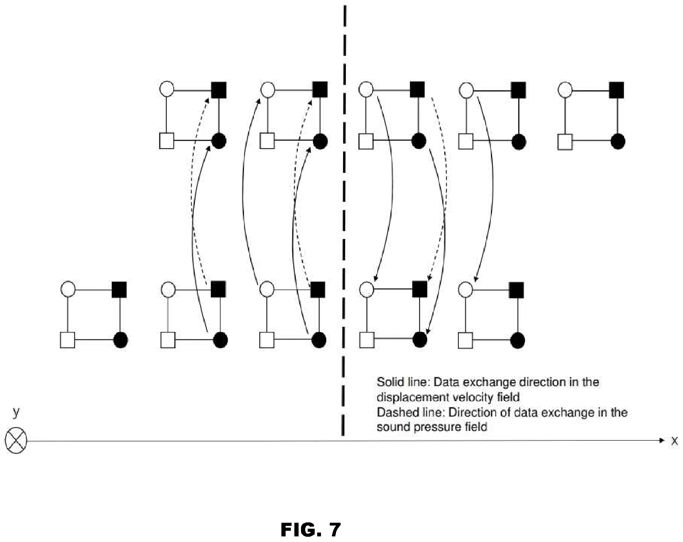

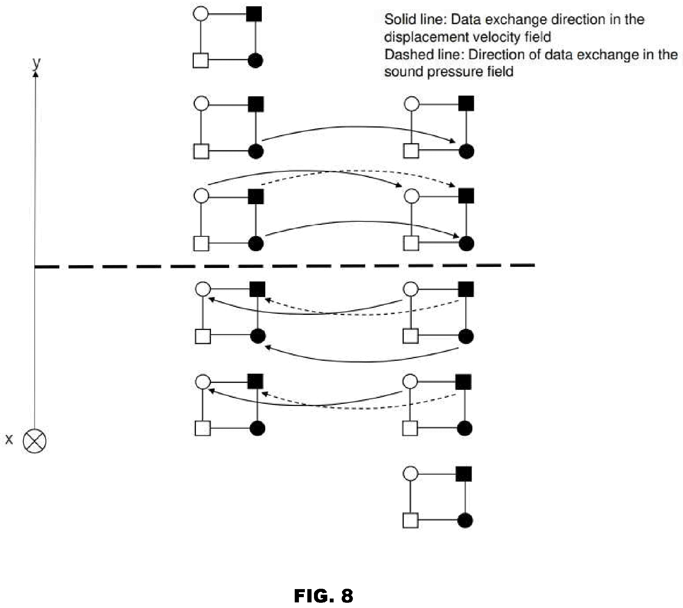

To accelerate computation, the computational domain is partitioned into subdomains, which are assigned to multiple computer units at the division boundaries shown in FIG. 6. FIG. 6 illustrates examples ranging from no partitioning to partitioning into sixteen subdomains. The number of subdomains can be selected as desired according to the performance of the CPUs and the network bandwidth. FIGS. 7 and 8 are diagrams illustrating data exchange between CPUs.

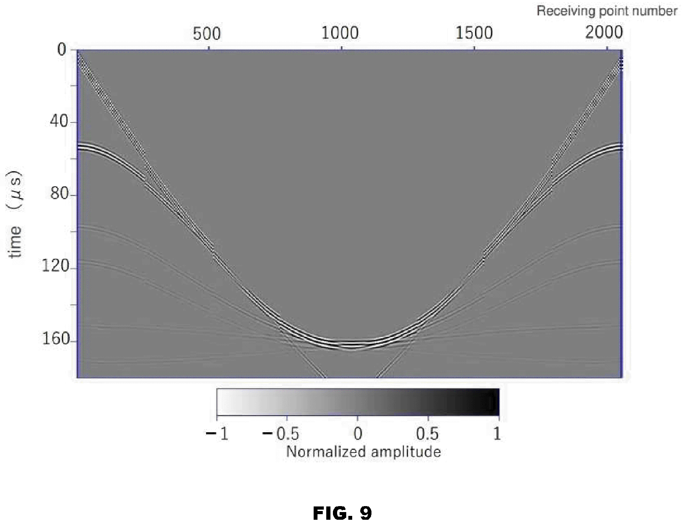

FIG. 9 is a diagram illustrating the time-series sound-pressure data received by 2,048 piezoelectric elements.

Next, a method for performing imaging by full-waveform inversion from simulated observed waveforms is described. For illustration, the numerical models shown in FIGS. 1 and 2 are used as the unknown target, and the simulated observed waveforms are those shown in FIG. 8. Because full-waveform inversion requires an initial model, the initial acoustic-velocity distribution is obtained using travel-time tomography. Because viscous attenuation Q-values cannot be obtained from travel-time tomography, the initial model is constructed using only water and fat, excluding the tumor and skin shown in FIG. 2. The density is fixed to the value of water and is not treated as an inversion parameter in the full-waveform inversion.

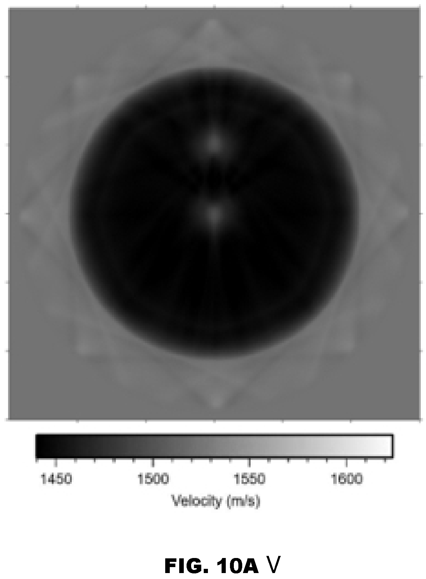

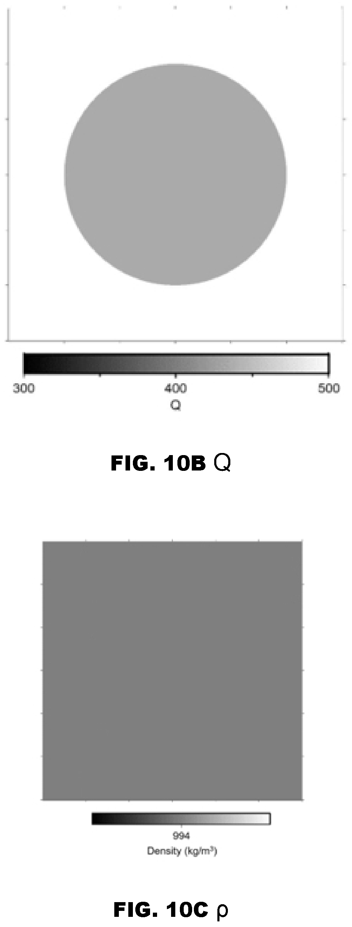

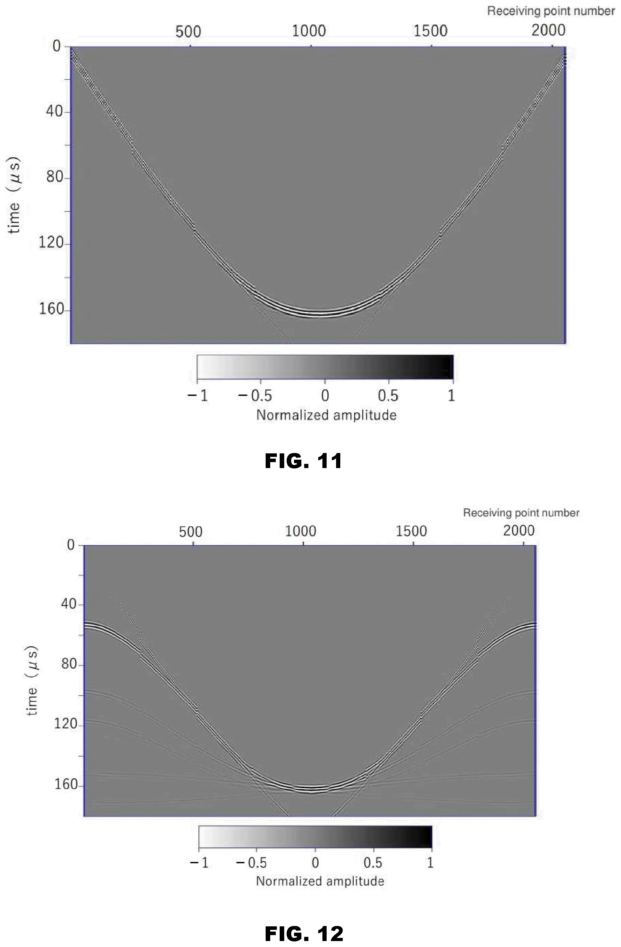

FIG. 10 illustrates the initial model obtained using travel-time tomography. Computing the forward-propagated wavefield using the initial model yields synthetic observation data at the observation points (FIG. 11), which can be compared with the observed data shown in FIG. 9.

FIG. 12 shows the residuals between the observed data obtained using the true model (FIG. 9) and the synthetic data obtained from the initial model (FIG. 11). In the initial model, direct waves can be synthesized in a manner consistent with the observed data when no body tissue lies between the transmitting and receiving points. In contrast, because the initial model does not include distinct tissue boundaries, reflected waves are scarcely reproduced.

The residual is computed in advance using Equation (5) below as the sum of the squared residuals between the observed data *(d_obs)* and the synthetic data *(d_syn)* calculated from the initial model, taken over all receiving points and all time samples.
Equation (5) ${f}_{c} = 1 / \left(2Δt\right) {\sum }_{s = 1}^{Ns} {\sum }_{r = 1}^{Nr} {\sum }_{t = 1}^{Nt} {\left[d_obs\left(s, r, t\right)-d_syn\left(s, r, t\right)\right]}^{2}$ (5) *Ns, Nr,* and *Nt* denote, respectively, the number of source (transmitting) points, the number of receiving points, and the number of time samples. The scalar fc serves as the objective function to be minimized by a quasi-Newton method described later, and is therefore also referred to as the cost function. Because the cost function must represent the entire computational domain, the values computed in each partitioned subdomain are collected and integrated, and the unified cost-function value is shared across all computing units.

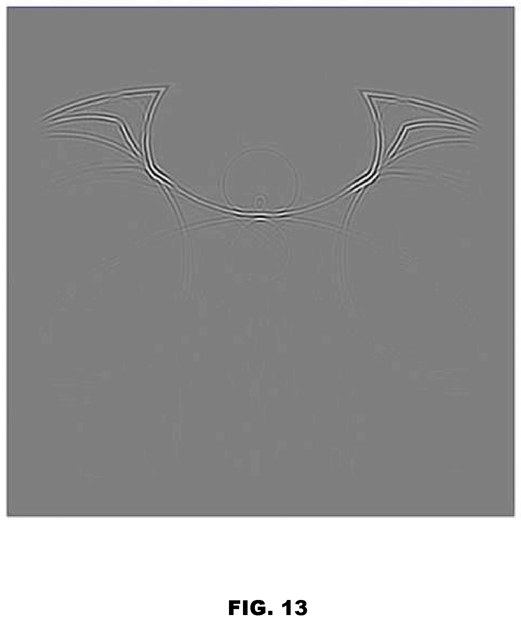

The residuals of the observed waveforms are back-propagated. A snapshot of the resulting back-propagated wavefield, taken 64 µs after the transmission time, is shown in FIG. 13. A time-domain cross-correlation is computed, at each imaging point, between (i) the forward-propagated wavefield obtained using the initial model and (ii) the wavefield generated by back-propagating the residuals of the observed waveforms. The zero-lag value of this cross-correlation constitutes the gradient of the model update in full-waveform inversion. Instead of storing the forward-propagated wavefield in memory, the overall model-update gradient is obtained more efficiently by reconstructing the forward-propagated wavefield backward in time, as described in Non-Patent Document 5. At the same time, a pseudo-Hessian matrix is also computed.

The present invention involves imaging of the viscous attenuation Q-value in addition to acoustic wave propagation velocity. To achieve this, the total model-update gradient must be decomposed into separate update gradients for the acoustic wave propagation velocity and the viscous attenuation Q-value. This decomposition method is not disclosed in Non-Patent Documents 1 or 2. Accordingly, four of the inventors subsequently described this method in Non-Patent Document 6.

To highlight this technical feature-namely, performing full-waveform inversion in the time domain instead of the frequency domain commonly used in prior techniques-the above processes (forward propagation, back propagation, and gradient computation) are presented together with the following comparative examples.

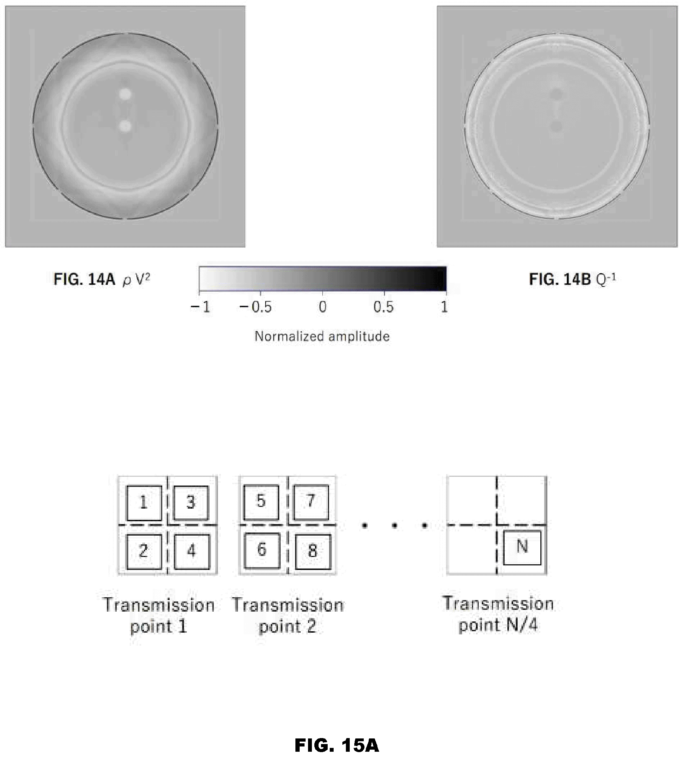

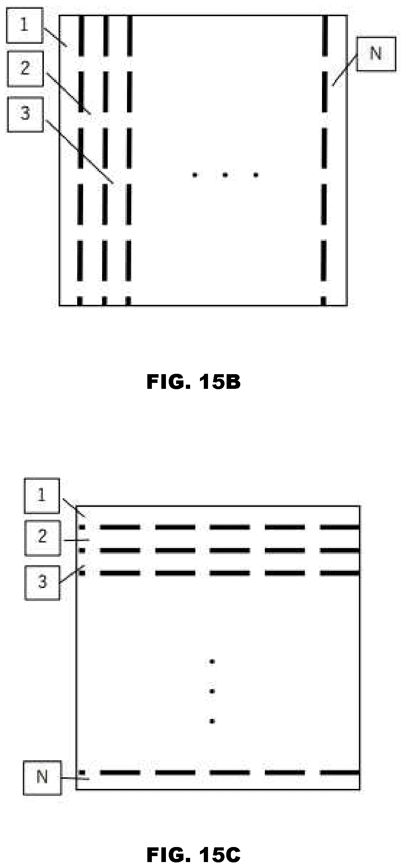

Once the update gradients of the acoustic wave propagation velocity and the viscous attenuation Q-value have been obtained, a Gaussian filter (smoothing) is applied to each of these gradients as well as to the pseudo-Hessian matrix. Because Gaussian smoothing cannot be performed while the domain is partitioned in both the x- and y-directions, the computational domain is repartitioned only in the x-direction, and Gaussian smoothing is then applied in the y-direction. Next, the computational domain is repartitioned in the y-direction, and Gaussian smoothing is applied in the x-direction (see FIG. 15). Because of the properties of Gaussian smoothing, reversing the order of the x- and y-direction smoothing yields the same result.

After Gaussian smoothing is applied, the update gradients (G) are preconditioned by applying the inverse pseudo-Hessian matrices (H⁻¹) corresponding to the acoustic wave propagation velocity and the viscous attenuation Q-value. This pseudo-Hessian-based correction improves the convergence of the update gradients by using only the diagonal components of the Hessian matrix-which dominate the influence on the gradient-without explicitly computing the full inverse Hessian, whose computation would otherwise be prohibitively expensive (see Non-Patent Document 7). However, this approach merely makes the computation numerically feasible; it is theoretically incomplete and is known to work largely on an empirical basis. This pseudo-Hessian-based correction is also expected to suppress the generation of locally erroneous images arising from geometric inhomogeneities.
Equation (6) $Δm = - {H}^{- 1} G$ (6)

By adding the model update *Δm* of Equation (6) to the initial model, a revised model is obtained. Because this revision is based on a linear approximation, the inversion must be iterated-typically tens to hundreds of times-using the updated model until the final image is achieved. In updating the model, convergence can generally be accelerated by applying a step length (α) to scale the update gradient. The procedure for updating the model from *mₖ* to *mₖ₊₁* during the k-th iteration is given by Equation (7)....
Equation (7) ${m}_{k + 1} = {m}_{k} + αΔ {m}_{k}$ (7)

Although some approaches (e.g., Patent Document 5) determine the step length solely from the cost function evaluated at the receiving points, it is fundamentally preferable to evaluate the entire system. Therefore, the step length is determined by using the scalar quantity obtained by dividing: the scalar product of the update gradient and the corrected update amount given in Equation (8), by the scalar value obtained by summing the squared components of the update gradient as defined in Equation (9).
Equation (8) ${\sum }_{i = 1}^{N} {H}_{i}^{- 1} {G}_{i}$ (8) Equation (9) ${\sum }_{i = 1}^{N} {G}_{i}^{2}$ (9) where *i* is the index of a grid point and *N* is the total number of grid points. The cost function is utilized solely for determining whether convergence has been achieved. Up to this point, the procedure corresponds to the steepest descent method, in which the step length α advances the model toward a minimum of the objective function along the steepest-descent direction. However, because the update gradient calculated in the time domain contains few local errors, a quasi-Newton method-specifically, the limited-memory Broyden-Fletcher-Goldfarb-Shanno (L-BFGS) method-can be employed. By updating the step length α along the tangential direction obtained from a quadratic approximation of the objective function, the convergence can be further accelerated.

However, when the number of iterations is *k* = 1, the model is updated using the steepest-descent method because no cost-function history is yet available. In addition, when the iteration number is k = 2, the model is updated by the conjugate gradient method. Thus, starting from iteration k = 3, the LBFGS method has access to the minimum requirement of two cost-function evaluations and two model-update vectors.

The domain assigned to each computing unit for iterative inversion may remain partitioned in the y-direction after Gaussian smoothing. In the model-update computation that applies this inversion technique, the process is carried out by comparing (i) the sum of the squares of the update gradient and (ii) the product of the update gradient and the corrected update amount, with their respective histories of previous iterations. Accordingly, the partial L2 norms computed in the y-partitioned subdomains must be integrated so that the resulting L2-norm value is shared across all computational units.

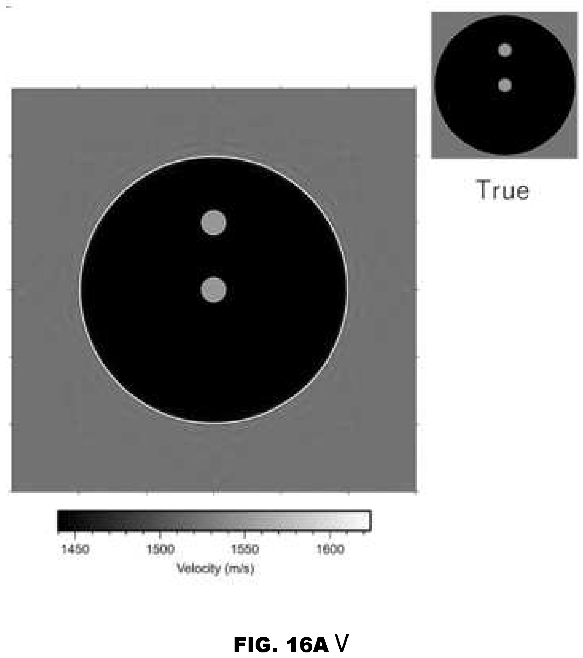

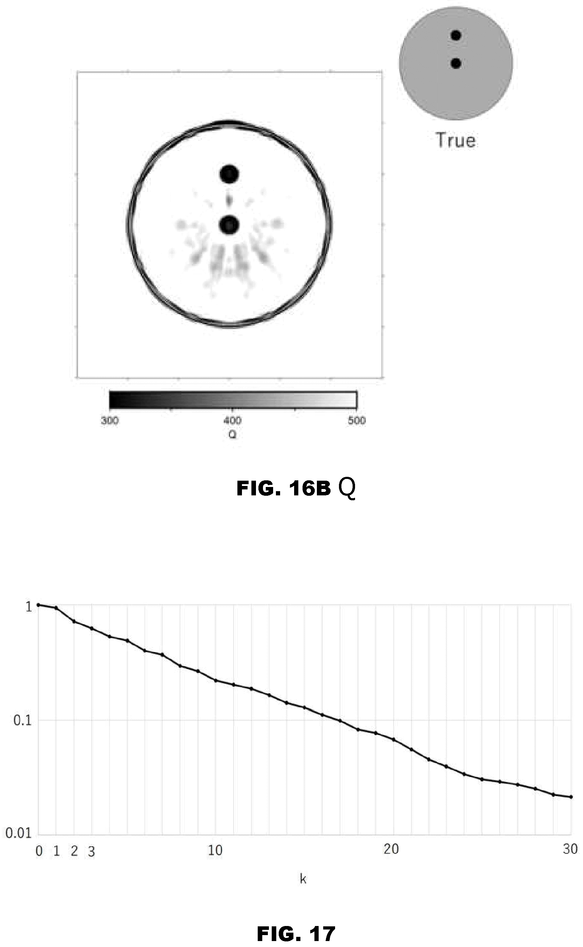

The imaging results obtained through the above-described procedure are shown in FIG. 16.

The cost function is plotted for the number of iterations in FIG. 17. It is observed that the cost function decreases as the number of iterations increases.

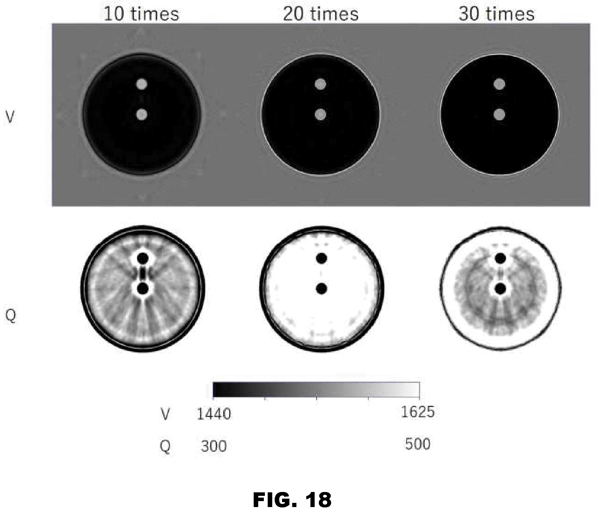

FIG. 18 shows the updated models after 10, 20, and 30 iterations. The tumor region is resolved within approximately 10 iterations, whereas the skin region requires about 20 to 30 iterations to be brought into focus.

The acoustic wave propagation velocity is imaged with high accuracy. The viscous attenuation Q-values contain a certain level of error. One source of this estimation error is the assumption of a constant density model. However, most of this estimation error arises because the image exhibits oscillations-particularly around the skin region-which indicates that the wavelength of the acoustic waves is too short relative to the size of the target. However, the relative variations in the viscous attenuation Q-value image faithfully preserve those of the true model, and the accuracy is sufficient for diagnostic imaging purposes.

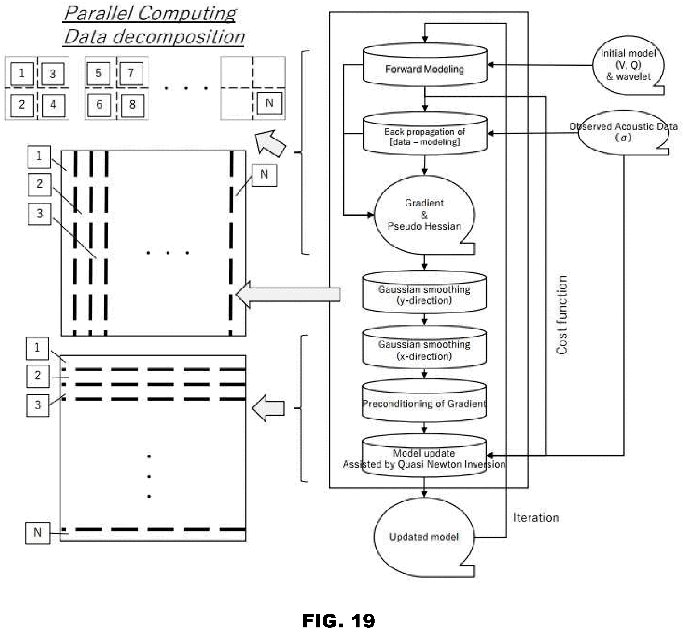

FIG. 19 illustrates, in the form of a flowchart, the domain-decomposition method of the present invention in accordance with the workflow of full-waveform inversion.

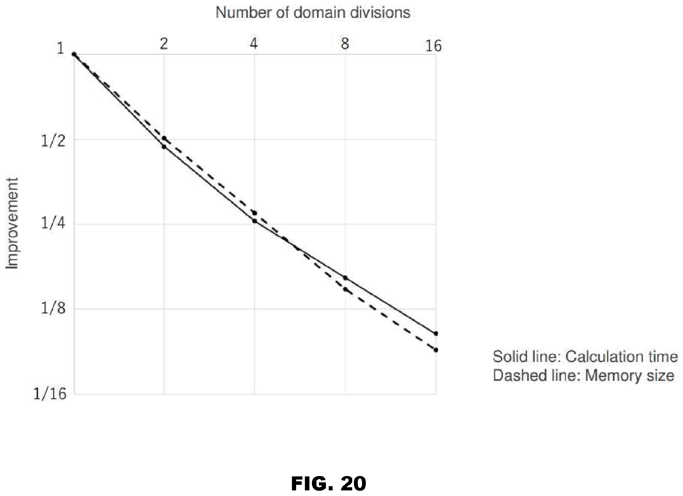

FIG. 20 shows benchmark-test results for computation time and memory usage. The benchmark test uses 3,005 pixels in the x-direction, 3,005 pixels in the y-direction, 5,625 time samples, and 24 transmitting points. The computing system consisted of a PC cluster of 24 servers connected via a LAN cable. Each server was equipped with two Intel Xeon E5-2690 v4 CPUs (2.6 GHz, 14 cores each) and 256 GB of memory. Values for parallel counts of 24, 48, 96, 192, and 384 are presented, normalized with respect to the value at a parallel count of 24. This corresponds to domain partition numbers of 1, 2, 4, 8, and 16, respectively, during the finite-difference calculation. When the number of domain partitions is 2, the computation time is reduced to less than half, as the efficiency gained within each computing unit due to reduced memory usage outweighs the loss caused by inter-unit communication. When the number of domain partitions is 2 or 4, the lengths of the domain boundaries that require communication are uniform across all computing units. In contrast, when the number of partitions is 8 or 16, the computing units responsible for the central subdomains have longer boundary lengths that require communication. It can be seen that the loss caused by this heterogeneity in communication volume becomes significant. However, both computation time and memory usage can be reduced almost in proportion to the degree of parallelization.

### Comparative Example

### Full-waveform Inversion in the Frequency Domain

The differences between performing full-waveform inversion in the time domain and in the frequency domain are described below.

The update gradient was calculated for the frequency-domain full-waveform inversion using the five frequency samples (251,770; 375,748; 501,633; 625,610; 751,495 Hz) shown in FIG. 3B.

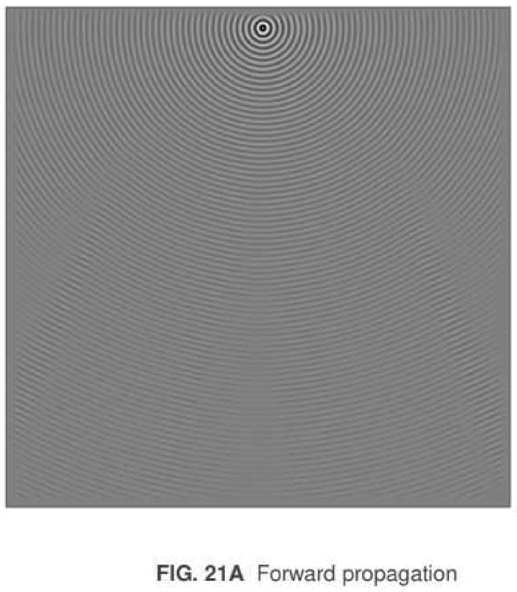

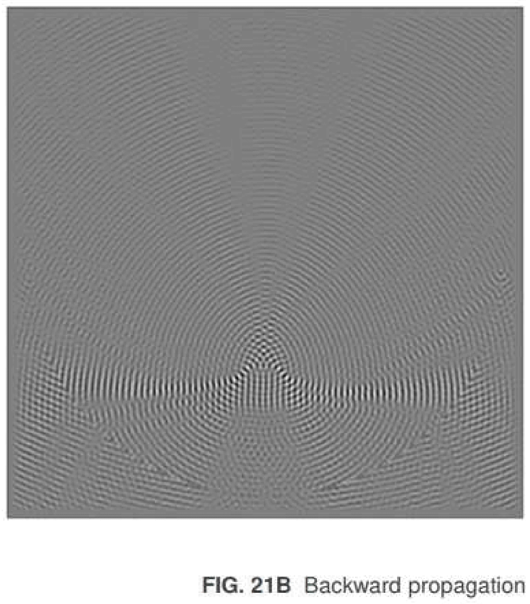

FIG. 21 shows the wavefield in the frequency domain (cosine-transform domain) at a frequency of 501,633 Hz. Because the wavefield in the frequency domain corresponds to a single-frequency oscillatory response, the wavefield exhibits a striped pattern.

Notably, strong responses appear outside the body tissue as well as outside the circle of the T/R point arrangement. However, back propagation was performed only for the records of receiving points located within a 90° aperture from the transmitting point. This is because the update-gradient results deteriorated when back propagation was performed using all receiving-point records within a 180° aperture.

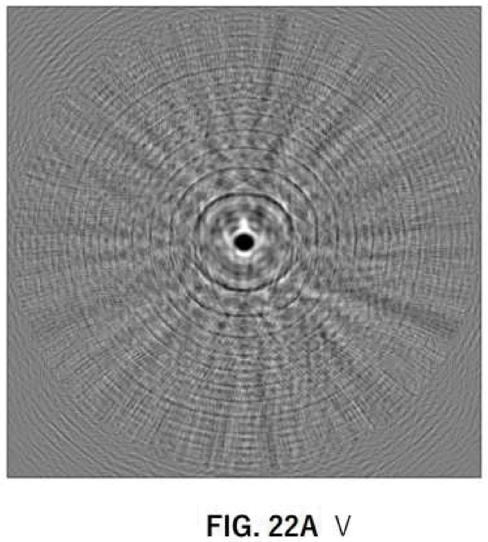

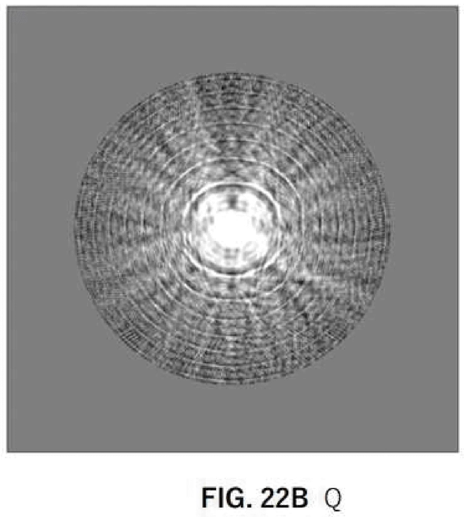

FIG. 22 shows the update gradients in the frequency domain. In the frequency domain, the update gradient is given by the real part of the complex product of the forward- and back-propagated wavefields; however, large gradient values remain outside the body tissue and even outside the circle of the T/R point arrangement. This exhibits a completely different behavior from the update gradients obtained in the time domain, as shown in FIG. 14. This is the long-standing cycle-skipping problem in full-waveform inversion. Various smoothing and regularization techniques have been proposed to mitigate this problem, but no effective solution was found in the inventors' trials.

Within the body tissue, the update gradient also exhibits periodic increases, forming a concentric, ring-like pattern. This corresponds to the period in which the phases of the five frequency samples used become aligned. Unfortunately, even if the frequency samples are varied within this range, periods of phase alignment-constructive or destructive-will inevitably occur because the wavelength (about 3 mm) is extremely short relative to the size of the target (16 cm). To avoid this, one would have to compute all frequency samples. However, this would in effect amount to carrying out full-waveform inversion in the time domain and would be significantly more computationally demanding than the time-domain computation itself.

The cycle-skipping problem can be mitigated by lowering the signal frequency band; however, this requires generating acoustic waves whose wavelengths correspond to 1 to 10 wavelengths relative to the size of the target. To mechanically generate low frequencies, the piezoelectric element would need to be made larger; however, this runs counter to one of the key advantages of ultrasound medical devices-providing low-cost diagnostic equipment.

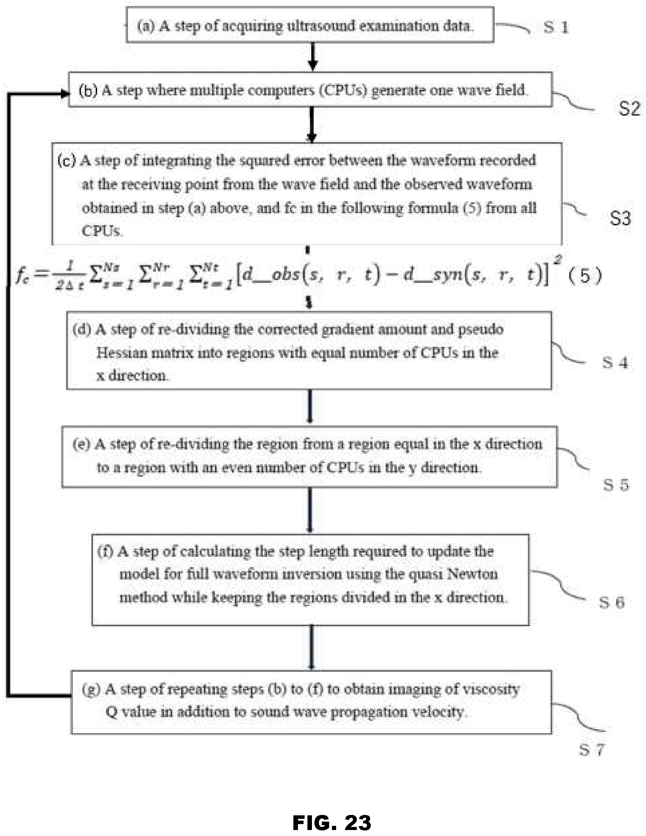

There is also a secondary wavefield arising from the gaps between segments. It can also be seen that the update gradient responds strongly to this artificially generated noise. Thus, although some Patent Documents state that full-waveform inversion can be implemented in both the frequency domain and the time domain, such a description is inadequate for the frequency domain, because it is feasible only if all frequencies can be computed. For example, Patent Document 4 refers to full-waveform inversion as "characterization of the boundary region" and limits its use to augmenting X-ray CT or travel-time tomography images used as the initial model. In general, features that are not imaged to a certain degree in the initial model cannot be imaged by full-waveform inversion. This implicitly indicates that the robustness of full-waveform inversion in the frequency domain is low. Thus, it can be concluded that robust imaging results can be obtained only in the time domain (see FIG. 23). In addition, it is believed that obtaining an image of the viscous attenuation Q-value together with the acoustic wave propagation velocity can improve the probability of tumor detection.

### Industrial Applicability

According to the present invention, a method is provided that enables fast and robust imaging of body tissue from ultrasound examination data using full-waveform inversion. This allows body tissue to be imaged more safely, at lower cost, and with higher resolution than X-ray CT or MRI used in existing examination systems. In particular, the present invention enables effective parallel computation of full-waveform inversion in the time domain across a large number of CPUs, thereby overcoming the conventional computation-time bottleneck and allowing analysis results to be obtained within a computation time comparable to that of existing examination systems. Accordingly, the present invention has high industrial applicability.

### Description of Reference Signs

T/R1: First transmitting/receiving point in the snapshot (FIG. 5)
T/R: Transmitting/receiving point
(A) Tumor
(B) Fat
(C) Skin
(D) Water
V: Acoustic wave propagation velocity
Q: Viscous attenuation Q-value
ρ: Medium density

## Claims

1. A method for imaging body tissue from ultrasound examination data using full-waveform inversion, comprising:
(a) acquiring ultrasound examination data;
(b) generating a single wavefield using a plurality of computing units (CPUs);
(c) integrating, across all of the CPUs, squared residuals between waveforms recorded at receiving points from the wavefield and observed waveforms acquired in step (a), the squared residuals corresponding to fc in Equation (5) below;
(d) repartitioning an update gradient and a pseudo-Hessian matrix in the x-direction into domains evenly assigned to the CPUs;
(e) repartitioning the domains that were evenly partitioned in the x-direction into domains evenly assigned to the CPUs in the y-direction;
(f) calculating a step length necessary for a model update in full-waveform inversion by a quasi-Newton method, while keeping the domains partitioned in the x-direction; and
(g) performing imaging of a viscous attenuation Q-value in addition to an acoustic wave propagation velocity by repeating steps (b) through (f).
Equation (5) ${f}_{c} = 1 / \left(2Δt\right) {\sum }_{s = 1}^{Ns} {\sum }_{r = 1}^{Nr} {\sum }_{t = 1}^{Nt} {\left[d_obs\left(s, r, t\right)-d_syn\left(s, r, t\right)\right]}^{2}$ (5) where *fc*, a scalar quantity, is the cost function defined as the sum of the squared residuals between the observed data (*d_obs*) and the synthetic data (*d_syn*) calculated from the initial model, taken over all receiving points and all time samples, and where *Ns*, *Nr*, and *Nt* denote, respectively, the number of source (transmitting) points, the number of receiving points, and the number of time samples.

2. The method according to claim 1, wherein, in step (b), data are exchanged at each time step, wherein the number of partitioned domains is set according to a computer system environment including a CPU count and a memory size, wherein the partitioned domains are applied to all three wavefield calculations, including forward propagation, back propagation, and forward-propagation rewinding calculations for full-waveform inversion, and wherein the update gradient and the pseudo-Hessian matrix are obtained while keeping the partitioned domains.

3. The method according to claim 1, wherein, after step (d), a Gaussian filter is applied in the y-direction, and wherein, after repartitioning the domains that were evenly partitioned in the x-direction into domains evenly assigned to the CPUs in the y-direction, a Gaussian filter is again applied in the y-direction.

4. The method according to claim 3, wherein the scalar sum of the squared components of the update gradient calculated in step (f), as defined in Equation (9) below, and the scalar product of the update gradient and the update amount corrected by the pseudo-Hessian matrix, as defined in Equation (8) below, are integrated across all of the CPUs.
Equation (8) ${\sum }_{i = 1}^{N} {H}_{i}^{- 1} {G}_{i}$ (8) Equation (9) ${\sum }_{i = 1}^{N} {G}_{i}^{2}$ (9) where *i is* the index of a grid point, *N* is the total number of grid points, *H⁻¹* is the inverse of the pseudo-Hessian matrix, and G is the update gradient.

5. The method according to claim 4, wherein the ultrasound examination data comprise ultrasound breast-cancer examination data, and wherein the body tissue comprises skin, mammary glands, and cancerous tissue.
